(19) Europäisches Patentamt
European Patent Office
Office européen des brevets

(11) **EP 2 175 867 B1**

(12) **EUROPÄISCHE PATENTSCHRIFT**

(45) Veröffentlichungstag und Bekanntmachung des Hinweises auf die Patenterteilung:
**30.10.2019 Patentblatt 2019/44**

(21) Anmeldenummer: **08785269.5**

(22) Anmeldetag: **31.07.2008**

(51) Int Cl.:
*A61K 33/00* *(2006.01)*          *A61K 33/10* *(2006.01)*
*A61M 1/16* *(2006.01)*          *B01F 5/10* *(2006.01)*

(86) Internationale Anmeldenummer:
**PCT/EP2008/006328**

(87) Internationale Veröffentlichungsnummer:
**WO 2009/018961 (12.02.2009 Gazette 2009/07)**

(54) **VERFAHREN UND VORRICHTUNG ZUM KONSTANTHALTEN EINES PH-WERTES EINER MEDIZINISCHEN FLÜSSIGKEIT BEIM ABLASSEN AUS EINEM BEHÄLTER**

METHOD AND DEVICE FOR MAINTAINING A CONSTANT PH VALUE OF A MEDICAL LIQUID DURING THE DISPENSING THEREOF FROM A CONTAINER

PROCÉDÉ ET DISPOSITIF POUR MAINTENIR CONSTANT LE PH D'UN LIQUIDE MÉDICAL LORSQU'IL S'ÉCOULE D'UN RÉCIPIENT

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **07.08.2007 DE 102007037099**

(43) Veröffentlichungstag der Anmeldung:
**21.04.2010 Patentblatt 2010/16**

(73) Patentinhaber: **Fresenius Medical Care Deutschland GmbH**
**61352 Bad Homburg (DE)**

(72) Erfinder: **RAUCH, Achim**
**67746 Langweiler (DE)**

(74) Vertreter: **Laufhütte, Dieter et al**
**Lorenz Seidler Gossel**
**Rechtsanwälte Patentanwälte**
**Partnerschaft mbB**
**Widenmayerstraße 23**
**80538 München (DE)**

(56) Entgegenhaltungen:
EP-A- 0 491 981          WO-A-03/086973
WO-A-2006/090869          DE-A1- 2 358 759

EP 2 175 867 B1

**Beschreibung**

Die vorliegende Erfindung betrifft ein Verfahren und eine Vorrichtung zum Konstanthalten eines pH-Werts einer medizinischen Flüssigkeit beim Ablassen aus einem Behälter. Ein solches Verfahren bzw. eine solche Vorrichtung kommt dabei insbesondere bei der Herstellung und Abfüllung von medizinischen Flüssigkeiten zum Einsatz, insbesondere bei der Herstellung und Abfüllung medizinischer Lösungen wie z. B. einer Bikarbonat-Dialyselösung.

Solche medizinischen Flüssigkeiten werden in so genannten Ansatzbehältern unter sterilen Bedingungen aus Wasser (WFI, Water for Injection) und den löslichen Zusatzstoffen hergestellt. Die Ansatzbehälter haben z. B. eine Größe von rund 24.000 Litern und sind mit veränderlichem Füllstand mit Flüssigkeit gefüllt. Über dem Flüssigkeitsspiegel befindet sich in dem Behälter ein Gasraum, der hinsichtlich des chemisch-physikalischen Gleichgewichts in komplexer Wechselwirkung mit der Lösung steht. Die medizinische Flüssigkeit, z. B. eine Bikarbonatlösung, soll dabei innerhalb sehr enger Toleranzen stets einen definierten pH-Wert aufweisen. Als Störgröße tritt dabei ein Ausgasen von $CO_2$ aus der Lösung in den Gasraum auf, was zu einer pH-Wert Erhöhung führt.

Um dem entgegen zu wirken, wird bei bekannten Verfahren die Lösung mit $CO_2$ begast, wobei das $CO_2$ über Düsen im Bereich des Bodens des Ansatzbehälters (Reaktors) in die Lösung eingedüst wird.

Ein solcher Ansatzbehälter ist aus der EP 491 981 A1 bekannt. Dabei wird während der Herstellung der Lösung CO2, welches während des Auflösens aus der Lösung austritt, über einen Gaskreislauf aus dem Gasraum abgepumpt und der Lösung durch Einperlen wieder zugeführt. Weiterhin kann zusätzlich CO2 mittels einer Gasflasche eingeperlt werden, um das Gleichgewicht zu verschieben.

Weiterhin ist aus der DE 2 358 759 A bekannt, über der Lösung im Gasraum eine gasförmige Schicht ausgebildet wird, welche aus CO2 und einem Inertgas besteht und eine solche Konzentration an CO2 aufweisen soll, dass der durch das Gleichgewicht innerhalb der Lösung gegebene Partialdruck ausgeglichen wird. Die Schutzschicht soll auch beim Abfüllen der Lösung aufrecht erhalten werden.

Aus der WO2006/090869 A1 ist es weiterhin bekannt, bei einer hypochlorige Säure oder Chlorsäure enthaltenden Lösung den pH-Wert durch Zugabe von CO2 zu einem Druckbehälter einzustellen. Dabei wird der pH-Wert über eine Sonde gemessen und die zugegebene Menge an CO2 über diese Messung eingeregelt.

Jedoch wirkt sich erschwerend aus, dass pH-Wert-Sonden mit zunehmender Zeit driften bzw. Messwertabweichungen aufweisen und deshalb regelmäßig kalibriert werden müssen. Dies steht im Falle von Inline-Sonden jedoch im Widerspruch zur Aufrechterhaltung der sterilen Bedingungen. Dabei wird einem Anstieg des pH-Werts aufgrund des Ausgasens von $CO_2$ derart entgegen gewirkt, dass anhand von regelmäßig manuell zu ziehenden Lösungsproben mit anschließender Laborbestimmung des pH-Werts Abweichungen vom Sollwert festgestellt werden und daraufhin nach Erfahrungswerten die Lösung über Düsen am Reaktorboden mit $CO_2$ begast wird. Dadurch ergibt sich über die Herstelldauer ein typischer 2-Punktregler-Verlauf des pH-Werts. Die Regelabweichung des pH-Wertes ist, aufgrund des enormen Zeitbedarfs der Analytik, beträchtlich. Das Herstellverfahren ist dementsprechend träge. Zudem ist dieses Verfahren zur Aufrechterhaltung eines konstanten pH-Werts äußerst personalaufwendig und entzieht sich einer Automatisierung. Insbesondere wirkt sich erschwerend aus, dass pH-Wert-Sonden mit zunehmender Zeit driften bzw. Messwertabweichungen aufweisen und deshalb regelmäßig kalibriert werden müssen. Dies steht im Falle von Inline-Sonden jedoch im Widerspruch zur Aufrechterhaltung der sterilen Bedingungen.

Aufgabe der vorliegenden Erfindung ist es daher, ein Verfahren und eine Vorrichtung zum Konstanthalten eines pH-Werts einer medizinischen Flüssigkeit beim Ablassen aus einem Behälter zur Verfügung zu stellen, welches einfacher, zeitsparender und kostengünstiger ist. Ebenso ist es Aufgabe der vorliegenden Erfindung, eine Automatisierung des Verfahrens zum Aufrechterhalten eines konstanten pH-Wertes einer medizinischen Flüssigkeit beim Ablassen aus einem Behälter zu ermöglichen.

Erfindungsgemäß wird diese Aufgabe von einem Verfahren zum Konstanthalten eines pH-Werts einer medizinischen Flüssigkeit beim Ablassen aus einem Behälter gemäß Anspruch 1 gelöst. Bei diesem Verfahren strömt erfindungsgemäß während des Ablassens der medizinischen Flüssigkeit $CO_2$ und mindestens ein weiteres Gas oder Gasgemisch in den Behälter zu. Die vorliegende Erfindung macht sich dabei die Erkenntnis zu Nutze, dass Flüssigkeit und Gasraum im Behälter in enger Wechselwirkung stehen. Dabei gast bei einem unterhalb der Gleichgewichtsbedingungen liegenden $CO_2$-Partialdruck $CO_2$ solange unter Erhöhung des pH-Werts aus der Flüssigkeit aus, bis die Flüssigkeit und darüber liegender Gasraum im chemisch-physikalischen Gleichgewicht stehen. Umgekehrt wird bei einem oberhalb der Gleichgewichtsbedingungen liegenden $CO_2$-Partialdruck im Gasraum $CO_2$ von Flüssigkeit absorbiert, was zu einer pH-Wert Erniedrigung führt. Entscheidend für das physikalisch-chemische Gleichgewicht ist dabei der Anteil an $CO_2$ in der Gasraumatmosphäre im Bezug auf den pH-Wert der Flüssigkeit, unter Berücksichtigung physikalisch-chemischer Randbedingungen wie des Drucks und der Temperatur im Gasraum und in der Lösung. Bei einem Ablassen der Flüssigkeit durch die Absenkung des Flüssigkeitsspiegels und die damit einhergehende Vergrößerung des Gasraums über der medizinischen Flüssigkeit ändern sich nun die physikalischen Randbedingungen des chemisch-physikalischen Gleichgewichts zwischen Flüssigkeit und Gasraum, was bei dem oben beschriebenen bekannten Verfahren zu der pH-Wert Verschiebung führt, welche dann nachträglich korrigiert werden muss. Durch das Zuströmen von $CO_2$ und mindestens

eines weiteren Gases oder Gasgemisches gemäß der vorliegenden Erfindung ist es nun dagegen möglich, durch eine entsprechende Zuführung von $CO_2$ und mindestens einem weiteren Gas oder Gasgemisch trotz der sich ändernden physikalischen Randbedingungen des Gasraums beim Ablassen der Flüssigkeit das ursprünglich eingestellte bzw. erreichte chemisch-physikalische Gleichgewicht aufrecht zu erhalten, so dass es erst gar nicht zu einer Verschiebung des pH-Werts kommt. Das Zuströmen des mindestens einen weiteren Gases oder Gasgemischs neben $CO_2$ ist dabei entscheidend, um die Gleichgewichts-Bedingungen erfüllen zu können. Das weitere Gas oder Gasgemisch sollte dabei vorteilhafterweise zumindest bezüglich des $CO_2$ nicht nennenswert mit der Flüssigkeit reagieren bzw. nur kleine oder vernachlässigbare Mengen an $CO_2$ enthalten. Insbesondere kann dabei als weiteres Gasgemisch Luft zum Einsatz kommen.

Das erfindungsgemäße Verfahren hat den Vorteil, dass der pH-Wert auf einfache Weise konstant gehalten werden kann, wobei Schwankungen bereits von vornherein verhindert werden, anstatt sie im nachhinein auszugleichen. Damit ergibt sich eine Konstanthaltung des pH-Werts bei einfacherem und schnellerem Verfahrensablauf, welcher sich zudem automatisieren lässt.

Das erfindungsgemäße Verfahren hat den weiteren Vorteil, dass hierdurch die ohnehin unbefriedigende Methode zur Einhaltung des pH-Sollwerts über entsprechendes Ziehen von Lösungsproben mit anschließender Laborbestimmung des pH-Werts durch eine einfache Methode ersetzt werden kann, bei der gar keine pH-Wert Analytik in der Lösung erforderlich ist. Insbesondere ist eine pH-Wert Bestimmung nur noch einmalig nach Abschluß des Herstell- und Abfüllvorgangs zur abschließenden gesetzlich geforderten Qualitätskontrolle der Lösung erforderlich.

Erfindungsgemäß wird bei dem erfindungsgemäßen Verfahren weiterhin eine Änderung des Füllstands der Flüssigkeit gemessen und anhand der Messung die Menge an $CO_2$, welche in den Behälter zugegeben wird, bestimmt. Die Änderung des Füllstands läßt sich dabei z. B. über einen Radarsensor berührungslos messen. Die Änderung des Füllstands als Steuergröße für die Zugabe von $CO_2$ ermöglicht eine einfache und dennoch effektive Steuerung der $CO_2$-Zuführung.

Vorteilhafterweise wird bei dem erfindungsgemäßen Verfahren eine zuzugebende Menge an $CO_2$ bestimmt und anhand der Bestimmung kontrolliert zugegeben. Insbesondere kann so die Menge an $CO_2$, welche beim Ablassen der Flüssigkeit zur Aufrechterhaltung des pH-Werts in der Flüssigkeit nötig ist, berechnet und kontrolliert zugegeben werden. Dabei läßt sich der pH-Wert der Lösung durch die Aufrechterhaltung des chemisch-physikalischen Gleichgewichts zwischen Flüssigkeit und Gasraum durch die kontrollierte Zuführung von $CO_2$ anhand der Berechnung konstant halten. Insbesondere muss dabei die zur Aufrechterhaltung des chemisch-physikalischen Gleichgewichts im Bezug auf das ebenfalls zuströmende mindestens eine weitere Gas oder Gasgemisch benötigte $CO_2$-Menge bestimmt werden.

Weiterhin vorteilhafterweise wird bei dem erfindungsgemäßen Verfahren eine Änderung des Gasraumvolumens im Behälter über der Flüssigkeit bestimmt, insbesondere anhand der oben beschriebenen Änderung des Füllstands der Flüssigkeit, wobei die zuzugebende Menge an $CO_2$ in Abhängigkeit von dieser Änderung des Gasraumvolumens bestimmt wird. Hierdurch kann berücksichtigt werden, dass sich die physikalischen Randbedingungen des Gasraums durch die Änderung des Gasraumvolumens ändern, so dass zur Aufrechterhaltung des chemisch-physikalischen Gleichgewichts zwischen Gasraum und Flüssigkeit eine entsprechende $CO_2$-Menge zugegeben werden muß.

Weiterhin vorteilhafterweise strömt bei dem erfindungsgemäßen Verfahren Umgebungsluft über einen Sterilfilter nach, so dass bei einer Änderung des Füllstands der Flüssigkeit der Druck im Gasraum dem Umgebungsdruck selbsttätig angeglichen wird. Die Umgebungsluft stellt damit das mindestens eine Gasgemisch dar, welches zusammen mit dem $CO_2$ in den Behälter zuströmt, wenn medizinische Flüssigkeit aus dem Behälter abgelassen wird. Durch das Nachströmen über den Sterilfilter ist so für einen automatischen Druckausgleich gesorgt, ohne dass hier eine komplizierte Regelung oder Steuerung nötig wäre, so dass die Aufrechterhaltung des physikalisch-chemischen Gleichgewichts vereinfacht wird. Der Sterilfilter ist dabei zwingend notwendig, um eine Kontamination der Flüssigkeit zu verhindern. Ein besonders einfaches Vorgehen ergibt sich dabei insbesondere in Kombination mit einer kontrollierten Zugabe von $CO_2$, welche den Zufluß von Außenluft mit niedrigem $CO_2$-Gehalt über den Sterilfilter ausgleicht und so das physikalisch-chemische Gleichgewicht aufrecht erhält.

Weiterhin vorteilhafterweise wird bei dem erfindungsgemäßen Verfahren das $CO_2$ in einer solchen Menge zugegeben, dass ein rechnerisch bestimmter mittlerer $CO_2$ Volumenanteil im Gasraum konstant bleibt. Untersuchungen des Erfinders haben dabei ergeben, dass eine solche Zugabe von $CO_2$ auf Grundlage eines rechnerisch bestimmten mittleren $CO_2$ Volumenanteils im Gasraum hervorragend dazu geeignet ist, den pH-Wert der Flüssigkeit über den gesamten Ablassprozess konstant zu halten. Eine Messung des $CO_2$ Volumenanteils wäre dagegen kaum möglich bzw. sinnvoll, da der $CO_2$ Partialdruck bzw. $CO_2$ Volumenanteil im Gasraum lediglich eine theoretische Größe darstellt, welche sich im Gasraum eines Mischgases jedoch nicht sinnvoll messen läßt, weil in der Praxis im Gasraum aufgrund der unterschiedlichen Gasdichten eine Entmischung und Konzentrationsgradientenbildung auftritt. Dementsprechend ließe sich zu der zur Einhaltung des chemisch-physikalischen Gleichgewichts erforderliche theoretische $CO_2$-Partialdruck kaum sinnvoll überprüfen. Durch die Zugabe von $CO_2$ anhand des rechnerisch bestimmten mittleren $CO_2$-Volumenanteils im Gasraum wird in der vorliegenden Erfindung eine solche Messung des $CO_2$-Partialdrucks dagegen überflüssig. Die Untersuchungen der Erfinder haben vielmehr ergeben, dass der pH-Wert der Flüssigkeit konstant gehalten werden kann, indem der Vergrößerung des Gasraums durch eine entsprechende Zugabe von $CO_2$ Rechnung getragen wird.

Weiterhin vorteilhafterweise wird bei dem erfindungsgemäßen Verfahren die zuzugebende Menge an $CO_2$ auf Grundlage eines rechnerisch ermittelten chemisch-physikalischen Gleichgewichts zwischen der Flüssigkeit und dem darüber liegenden Gasraum bestimmt. Anhand des gewünschten pH-Wertes der Flüssigkeit und weiterer Randbedingungen wie der Temperatur und dem Druck kann dabei anhand eines rechnerisch ermittelten chemisch-physikalischen Gleichgewichts insbesondere der notwendige $CO_2$-Volumenanteil des Gasraums im Voraus bestimmt werden. Dieser kann dann erfindungsgemäß konstant gehalten werden, um auch den pH-Wert in der Flüssigkeit konstant zu halten. Untersuchungen des Erfinders haben gezeigt, dass dieser rechnerisch ermittelte mittlere $CO_2$-Volumenanteil des chemisch-physikalischen Gleichgewichtszustandes aufgrund der unterschiedlichen Gasdichten der Gase und der damit verbundenen Gradientenbildung der Gaskonzentrationen im Gasraum nicht lokal gemessen werden kann. Dennoch wird bei Zugabe einer Menge $CO_2$, die rechnerisch zu dem ermittelten mittleren $CO_2$-Volumenanteil des Gleichgewichtszustandes führt, der pH-Wert in der Lösung konstant gehalten. Ebenfalls ist auch die Bestimmung und/oder Überprüfung der erfindungsgemäß zuzugebenden Menge an $CO_2$ durch Messreichen möglich, deren Ergebnisse dann in einem Speicher abgelegt werden können.

Vorteilhafterweise erfolgt dabei die Bestimmung der zuzugebenden Menge an $CO_2$, ohne dass während des Ablassens eine Messung des pH-Wertes der Flüssigkeit durchgeführt würde. Solche Messungen sind langwierig und verzögern und verteuern das Verfahren. Erfindungsgemäß kann auf eine solche Messung wie oben beschrieben verzichtet werden.

Vorteilhafterweise erfolgt dabei die Bestimmung der zuzugebenden Menge an $CO_2$, ohne dass während des Ablassens eine Messung der $CO_2$-Konzentration im Gasraum durchgeführt würde. Wie bereits oben beschrieben ist eine solche Messung ohnehin kaum möglich und sinnvoll durchzuführen. Erfindungsgemäß kann dabei, wie oben beschrieben, auf eine solche Messung verzichtet werden, insbesondere indem $CO_2$ in einer solchen Menge zugegeben wird, dass ein rechnerisch bestimmter mittlerer $CO_2$-Volumenanteil im Gasraum konstant bleibt.

Vorteilhafterweise strömt dabei bei dem erfindungsgemäßen Verfahren das $CO_2$ und das mindestens eine weitere Gas oder Gasgemisch während dem Abfüllen oberhalb des Flüssigkeitsspiegels in den Gasraum zu. Das $CO_2$ und das mindestens eine weitere Gas oder Gasgemisch fließt damit direkt in den Gasraum, um die Gasraumatmosphäre mit der Flüssigkeit im chemisch-physikalischen Gleichgewicht zu halten.

Weiterhin vorteilhafterweise wird jedoch vor dem Ablassen der Flüssigkeit eine vorher bestimmte Menge an $CO_2$ in die Flüssigkeit eingedüst. Insbesondere wird dabei erfindungsgemäß nach Abschluß des Ansatzprozesses aus Einfüllen von Wasser und Zugabe der löslichen Zuschlagstoffen, während welchen gar keine Maßnahmen zur Einstellung eines bestimmten pH-Wertes getroffen werden, sondern das Ausgasen von bereits etwaig vorhandenem $CO_2$ hingenommen wird, eine vorher aufgrund chemisch-physikalischer Berechnungen ermittelte Menge an $CO_2$ initial in die Lösung eingedüst. Vorteilhafterweise kommen dabei Injektordüsen zur Anwendung, die das feinperlige Eindüsen bei hohem Impulsaustausch zwischen Gas- und Flüssigphase ermöglichen. Hierdurch ergibt sich in der Lösung initial ein gewisser pH-Wert, wobei sich ein physikalisch-chemisches Gleichgewicht zwischen Flüssigkeit und darüber liegendem Gasraum einstellt. Hierfür kann vorteilhafterweise während des Eindüsens ein Ausströmen von Gas aus dem Gasraum unterbunden werden. Dieses chemisch-physikalische Gleichgewicht wird dann erfindungsgemäß gemäß dem Verfahren, wie es oben beschrieben wurde, beibehalten, um so auch den pH-Wert beizubehalten.

Vorteilhafterweise kommt das erfindungsgemäße Verfahren dabei beim Herstellen bzw. Ablassen einer Bikarbonatlösung zum Einsatz. Insbesondere bei einer solchen Bikarbonatlösung tritt das oben beschriebene Problem des Ausgasens von $CO_2$ aus der Lösung auf, so dass hier erfindungsgemäß der pH-Wert über die Aufrechterhaltung des physikalisch-chemischen Gleichgewichts konstant gehalten werden kann.

Die vorliegende Erfindung umfaßt weiterhin eine Vorrichtung zum Konstanthalten eines pH-Werts einer medizinischen Flüssigkeit beim Ablassen aus einem Behälter, wobei der Behälter einen Ablauf für die Flüssigkeit, mindestens einen Zulauf für $CO_2$ sowie einen Zulauf für mindestens ein weiteres Gas oder Gasgemisch aufweist. Erfindungsgemäß weist die Vorrichtung eine elektronische Steuerung zur Bestimmung und automatischen Zuführung einer $CO_2$-Menge zur anfänglichen Lösungseinstellung mittels der Gas-Flüssig-Düsen und/oder während des Ablassens der Flüssigkeit auf. Insbesondere erfolgt die Bestimmung und automatische Zuführung der $CO_2$-Menge dabei gemäß einem der oben beschriebenen erfindungsgemäßen Verfahren. Hierdurch ergeben sich die gleichen Vorteile, wie sie oben bezüglich des Verfahrens beschrieben wurden. Vorteilhafterweise weist der Behälter dabei jeweils einen Zulauf für $CO_2$ in die Flüssigkeit und für $CO_2$ in den Gasraum auf.

Weiterhin vorteilhafterweise weist die erfindungsgemäße Vorrichtung einen Messwertgeber zur Bestimmung des Füllstandes im Behälter auf, insbesondere über Mikrowellen (Radar). Über diesen Messwertgeber kann der Füllstand im Behälter bestimmt werden, so dass die zuzuführende $CO_2$-Menge anhand des Füllstandes und/oder der Füllstandsabnahme gesteuert werden kann.

Weiterhin vorteilhafterweise weist die erfindungsgemäße Vorrichtung eine Verbindung zwischen dem Gasraum des Behälters und der Umgebung auf, durch welche über einen Sterilfilter Umgebungsluft in den Gasraum zuströmen kann. Diese Verbindung stellt damit den Zulauf für das mindestens eine weitere Gas oder Gasgemisch dar und sorgt für einen selbsttätigen Druckausgleich sowie für ein Zuströmen von Umgebungsluft. Durch ein gleichzeitiges entsprechendes Zuführen von $CO_2$ über die elektronische Steuerung wird dabei das physikalisch-chemische Gleichgewicht aufrecht erhalten.

Weiterhin vorteilhafterweise umfaßt die Erfindung eine Vorrichtung zur Durchführung eines Verfahrens, wie es oben beschrieben wurde.

Die vorliegende Erfindung wird nun anhand von Zeichnungen sowie einem Ausführungsbeispiel näher dargestellt. Dabei zeigen:

Figur 1: eine Prinzipdarstellung des Behälters eines Ausführungsbeispiels der erfindungsgemäßen Vorrichtung,

Figur 2: den Verlauf des pH-Werts während des Ablassen unter Verwendung eines Verfahrens nach dem Stand der Technik,

Figur 3: den Verlauf des pH-Werts während des Ablassen unter Verwendung eines Ausführungsbeispiels des erfindungsgemäßen Verfahrens, und

Figur 4. ein Verlaufsdiagramm eines Ausführungsbeispiels der erfindungsgemäßen Steuerung.

In dem im folgenden dargestellten Ausführungsbeispiel der vorliegenden Erfindung wird das erfindungsgemäße Verfahren bzw. die erfindungsgemäße Vorrichtung dazu verwendet, den pH-Wert einer Bikarbonatlösung während des Abfüllens konstant zu halten. Dabei wird zunächst nach Abschluss des Ansatzprozesses der Bikarbonatlösung, während dessen gar keine Maßnahmen zu Einstellung eines bestimmten pH-Wertes getroffen werden, sondern das Ausgasen von $CO_2$ hingenommen wird, eine vorher aufgrund chemisch-physikalischer Berechnungen ermittelte Menge an $CO_2$ initial in die Lösung eingedüst. Im Ausführungsbeispiel werden beispielsweise in einem Ansatzbehälter mit 24.000 Litern Bikarbonatlösung mit 70 mmol/l Bikarbonat initial definiert 10,5 kg $CO_2$ feinperlig in die Lösung eingedüst. Dabei kommen Injektordüsen zur Anwendung, die das feinperlige Eindüsen ermöglichen.

Während des Ablassens der fertigen Lösung über eine Bodenöffnung des Ansatzbehälters zum Abfüllen der Lösung z. B. in Kunststoffbeutel muß nun erfindungsgemäß der pH-Wert der Lösung konstant gehalten werden. Dazu wird online in Abhängigkeit vom Füllstand eine individuell erforderliche Nachführbegasungsmenge $CO_2$ zur Eindüsung in den Gasraum berechnet. Hierbei wird berücksichtigt, dass beim Ablassen der Lösung die physikalischen Randbedingungen geändert werden, so dass es ohne eine erfindungsgemäße Nachführbegasung zu einer Verschiebung des chemisch-physikalischen Gleichgewichts zwischen Flüssigkeit und dem Gasraum kommen würde, was zu einem geänderten pH-Wert im abgefüllten Produkt führen würde.

Während des Entleervorgangs (Abfüllen des Produkts, Füllstandsabsenkung) wird in Abhängigkeit vom mittels Mikrowellen permanent gemessenen Füllstand der Bikarbonatlösung die nachzuführende $CO_2$-Menge berechnet und gerade soviel $CO_2$ in den Gasraum eingedüst, dass die $CO_2$-Menge im Gasraum einem berechneten theoretischen mittleren Volumenanteil von 20,5 Vol.-% $CO_2$ entspricht. Dieser aufgrund theoretischer Gleichgewichtsberechnung ermittelte Betrag läßt sich allerdings an keiner Stelle des Gasraums messen, weil es zu einer Gradientenbildung im Gasraum kommt. Eine $CO_2$-Messung ist erfindungsgemäß überhaupt nicht erforderlich, sondern es hat sich in Untersuchungen überraschend herausgestellt, dass die Einhaltung dieser Bedingungen ($x''_{CO2}$ = 20,5 Vol.-% bei 15°C und 1 bar) beim Entleeren zu einer Einhaltung des pH-Werts pH = 7,2 in der Bikarbonatlösung mit bisher nicht bekannter Genauigkeit auch über längere Zeiträume, bzw. über längere Entleervorgänge z. B. mehrere Tage, führt.

Darüber hinaus strömt zum Ausgleich des Unterdrucks bei Absenken des Füllstands Umgebungsluft über den Sterilfilter in den Gasraum. Wechselwirkungen zwischen der Luft bzw. dem $CO_2$-Anteil der Luft und der Bikarbonatlösung haben sich dabei als vernachlässigbar erwiesen. Bei geschlossenen Reaktoren ergäbe sich dagegen das Problem, dass sich die Gleichgewichtsbedingungen mit den Druckänderungen entsprechend ständig verschieben würden. Das erfindungsgemäße Verfahren hat dagegen den Vorteil, dass der Druck im Gasraum durch Nachströmen von Luft über den Sterilfilter konstant gehalten werden kann und das $CO_2$ nicht zum Druckausgleich zugegeben werden muss, sondern der Freiheitsgrad besteht, $CO_2$ unabhängig vom Druckausgleich definiert zuzugeben. Hierdurch kann erfindungsgemäß auch verhindert werden, dass zuviel $CO_2$ in den Gasraum eingedüst wird, was zu einer unerwünschten starken Absenkung des pH-Wertes mit der Ausbildung starker Gradienten für $x'_{CO2}$ unterhalb des Flüssigkeitsspiegels der Bikarbonatlösung führen würde.

Das Ausführungsbeispiel der vorliegenden Erfindung wird nun noch einmal im Detail näher erläutert.

Produkt - und Prozessbeschreibung

Zum Zwecke der Herstellung von sterilen Arzneimittel- oder auch Medizinproduktelösungen werden bestimmte Lösungstypen gemäß Ihrer einzustellenden Eigenschaften im Rahmen des Herstellprozesses mit Kohlendioxid begast.

Insbesondere geschieht dieses zur Einstellung eines bestimmten pH-Wertes. Dabei werden zuvor in destilliertem Wasser (WFI - water for injection) Hydrogencarbonat (Bikarbonat) oder auch andere geeignete Rohstoffe zugegeben und gelöst.

Im gegebenen Fall werden in automatisierten verfahrenstechnischen Anlagen im Großmaßstab (bis 24.000 Liter/ Chargengröße) Peritonealdialyselösungen hergestellt und in Kunststoffbeutelsysteme mit integrierten Schlauch- und Konnektorsystemen abgefüllt. Die Lösung wird dabei im abgefüllten Beutelsystem als Zweikammerlösung bis unmittelbar vor der Anwendung getrennt gelagert. Die separierten Lösungstypen in Kammer 1 bzw. Kammer 2 eines Beutelsystems werden als A-bzw. B-Lösung bezeichnet. Hierbei stellt die sogenannte B-Lösung die mit Kohlendioxid zu begasende Lösung dar.

Die Lösungen werden jeweils in entsprechend großen Behältern (Ansatztanks B1) aus nichtrostendem Stahl angesetzt, chemisch analysiert, u.U. korrigiert und sterilfiltriert in einen jeweils nachgeschalteten Gas-Flüssig Reaktor (Vorlage B2 bzw. B3). Eine Ansatzlinie besteht dabei jeweils aus einem Ansatztank und zwei Vorlagebehältern bzw. Gas-Flüssig Reaktoren.

Eine der Ansatzlinien dient dabei beispielsweise zur Herstellung und Bereitstellung der B-Lösung. Da die Ansatzlinie zwei sterile Vorlagebehälter B2 und B3 aufweist, werden die Ansätze zwar batchweise hergestellt, können aber quasikontinuierlich durch Wechsel bzw. Umschaltung der beiden Vorlagebehälter abgefüllt werden. Dabei befindet sich der jeweilige nicht am Füllprozess beteiligte Vorlagebehälter zwischenzeitlich in Reinigungs- und Sterilisationsphasen. Im Falle der bikarbonathaltigen B-Lösung kommen die beiden Vorlagebehälter, wegen der notwendigen Begasung der Lösung mit Kohlendioxid, jeweils einem Gas-Flüssig-Reaktor gleich.

Im Falle der B-Lösung erfolgt im Zustand der jeweiligen sterilen Vorlage die oben genannte Begasung mittels Kohlendioxid zur initialen pH-Wert Einstellung der Lösung. Diese findet damit nach dem Ansatzvorgang, der chemischen Analyse und der Sterilfiltration in eben diese Behälter statt.

Das Gas wird während des Prozesses der initialen pH-Wert Einstellung jeweils über ein im Bodenbereich der Behälter angebrachtes Verteilerrohr zugegeben. Die Versorgung mittels Kohlendioxid erfolgt aus einer mit den Behältern verrohrten Druckgasflasche. Die Druckgasflasche ist dezentral im Produktionsraum aufgestellt.

Nach Abschluss der Filtrationsprozesse und der initialen Einstellung des pH-Wertes werden die Lösungen A und B über die Dauer des Abfüllprozesses jeweils mittels Ringleitungen von ca. 100 m Länge im Kreislauf gefahren. Die beiden Ringleitungen führen von den Vorlagebehältern der Ansatzlinien zu den Füllereinheiten des örtlich entfernt liegenden Abfüllbereiches.

Über die jeweiligen Füller der Abfüllbereiche wird dabei quasikontinuierlich eine kleinere Teilmenge abgezogen und eine größere Menge permanent über ein Tauchrohr in den jeweiligen Tank zurückgeführt.

Die über jeweils mehrere Füllstellen der Füllereinheiten abgezogene Menge variiert dementsprechend zwischen wenigen 100 Litern pro Stunde bis weit über 1.000 Litern pro Stunde.

Die von den Füllereinheiten jeweils aus den Ringleitungen abgezogene Teilmenge wird über kurze Stichleitungen zu den einzelnen Füllstellen geführt.

Dementsprechend erfolgt eine unstetige Abnahme der Lösung aus der jeweiligen Ringleitung von A- und B-Lösung.

Zum Kompensieren der über die Pumpen eingetragenen Energie beziehungsweise der daraus resultierenden Erwärmung der Lösung, sind den Pumpen jeweils Wärmeübertrager nachgeschaltet. Damit kann auch über einen mehrtägigen Abfüllzyklus aus einer Vorlage die Temperatur konstant gehalten werden.

Die Ansatz- und Vorlagebehälter sind über hydrophobe Sterilfilter zur Umgebung, bzw. in den Produktionsraum, hin freigestellt.

Der Solltemperaturbereich der Lösung während der Abfüllung liegt zwischen 15°C und 20°C. Die Abfüllung der hier näher zu betrachtenden Doppelkammersysteme erfolgt vorzugsweise bei einer Temperatur von 15°C.

Im Falle dieser Lösungstypen ist die niedrigere Temperatur sinnvoll aufgrund der erhöhten Gaslöslichkeit von Kohlendioxid bei niedrigeren Temperaturen.

Aus Gründen des zeitlichen Ablaufes der Produktionsfolge und auf Grund der Notwendigkeit der Sterilisation der Vorlagebehälter bei jedem Chargenwechsel, lässt es sich nicht vermeiden neu angesetzte Lösungsmengen von etwa 15°C in einen noch sehr stark erwärmten Vorlagebehälter zu filtrieren. Dementsprechend ergeben sich durch solche Überlappungen kurzzeitige Unterdrücke (Kollabieren der restlichen Dampfmenge) aber auch Temperaturgradienten der steril filtrierten Lösung, soweit unmittelbar nach abgeschlossener Filtration (und nachgeschaltetem Filter-Integritätstest) der Wechsel auf die Folgecharge durchgeführt werden muss. Erst nach gewisser Zeit kann in solchen Fällen mit den bereits oben genannten integrierten Wärmeübertragern ein Temperaturniveau von 15°C erreicht werden.

Für die hier zu betrachtende B-Lösung der sogenannten Doppelkammerlösungen ist im folgenden die Zusammensetzung wiedergegeben. Als Einsatzstoff bei dem hier betrachteten Lösungstyp ist nur Natriumhydrogencarbonat vorgegeben.

Kohlendioxid ist dabei als Hilfsstoff zur pH-Wert - Einstellung deklariert. Die einzusetzende Menge wird später beschrieben.

**Tab. 1-1:** Deklaration der B-Lösung

| Deklaration | |
|---|---|
| **Kationen** | **Anionen** |
| Natrium 70,00 mmol/l | Hydrogencarbonat 70,00 mmol/l |

**Tab. 1-2:** Bestandteile der B-Lösung

| Bestandteile nach Deklaration | |
|---|---|
| **Rohstoff** | **Einwaage/ l** |
| Wasser für Injektionszwecke | 996,6 g |
| Natriumhydrogencarbonat Ph.Eur. | 5,881 g |
| Kohlendioxid entsprechend pH-Wert 7,2 - 7,3 | |

Im Falle des hier zu gewährleistenden pH-Wertes ist ein Bereich von 7,2 bis 7,3 pH-Wert-Einheiten sicherzustellen.

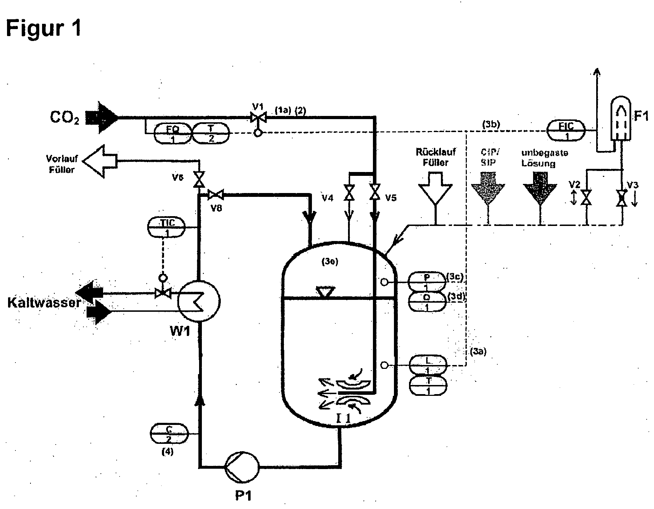

Figur 1 zeigt als Prinzipskizze die wesentlichen anlagentechnischen Zusammenhänge hinsichtlich des Absorptionsprozesses von Kohlendioxid in sterilen Arzneimittellösungen.

Erläuterungen zu Figur 1:

**Bezeichnungen**

CIP      cleaning in place; destilliertes Wasser 80°C, water for injection
SIP      sterilisation in place; Reindampf 121°C
$CO_2$      Kohlendioxid

**Apparate und Armaturen**

I1, I2, I3      Injektoren I1, I2, I3
BX      Reaktor B2 oder B3 als Sterilvorlage mit Injektoren I1, I2, I3
P1      Pumpe P1
W1      Wärmeübertrager W1
F1      Luftfilter F1 (hydrophober Sterilfilter PVDF)
V1      Ventil V1, Hauptzufuhr Kohlendioxid
V2      Ventil V2, Freistellung des Behälters zur Umgebung
V3      Ventil V3, Rückschlagventil zur Behälterbelüftung
V4      Ventil V4, Kohlendioxidnachführung in den Gasraum
V5      Ventil V5, Zufuhr $CO_2$ in Flüssigkeit
V6      Ventil V6, Vorlauf Abfüllung
V7      Ventil V7, Bypassventil Nachlösen von Kohlendioxid
V8      Ventil V8, "kleiner" Tankkreislauf

**Messtechnik**

FQ1/T2      Massendurchflussmessung $CO_2$
FIC1      Durchflussmessung nachströmender Raumluft

| TIC1 | Temperaturregelung über Durchfluss Kaltwasser |
|---|---|
| Q1 | Qualitätsgröße - $CO_2$-Gehalt der Dampfphase über der Flüssigkeitsoberfläche |
| Q2 | Qualitätsgröße - pH-Wert, Inline Messung |
| P1 | Druck/ Partialdruck $CO_2$ |
| L1 | Füllstand Behälter |
| T1 | Temperatur der Flüssigkeit |
| I1 | Injektor 1, Begasung der Lösung zur Einstellung des pH-Wertes |
| I2 | Injektor 2, Nachlösen von etwaigem überschüssigem Kohlendioxides des Gasraumes |
| I3 | Injektor 3, Mischen des Bypassstromes mit "vorgelöstem" Kohlendioxid aus Düse I2 |

## Begasungsprozess der Lösung vor Abfüllung

In der sogenannten B-Lösung befindet sich Bicarbonat (Natriumhydrogencarbonat) nach dem Ansetzen mit dem durch chemische Reaktion entstehenden Kohlendioxid in einem näherungsweisen thermodynamischen Gleichgewicht. Dabei ergibt sich ein pH-Wert gemäß den Messungen während des Ansatzvorganges im Ansatztank von ca. 8,3.

Die Lösung wird in einen der beiden Reaktoren B2 und B3 sterilfiltriert und in diesem zur Einstellung des pH-Wertes zusätzlich mit Kohlendioxid begast.

Die Begasung erfolgt dabei vorteilhafterweise über Injektordüsen.

## Einstellung des Endzustandes der Begasung

Der Begasungsvorgang erfolgte hinsichtlich des einzustellenden geminderten Druckes der Druckgasflasche und der Begasungsdauer zwischen den einzelnen Kontrollmessungen bei bekannten Verfahren willkürlich nach Erfahrungswerten des jeweiligen Bedienpersonals, wobei der Prozess noch durch etwaiges zeitgleiches Herunterkühlen der Lösung überlagert wird. Damit wird der gesamte Prozess nur sehr subjektiv erfasst.

Erst nach mehreren Begasungsunterbrechungen und jeweiligen pH-Wert-Messungen mit unveränderten Werten, konnte jeweils von einem repräsentativen pH-Wert-Status ausgegangen werden und der Begasungsvorgang weitergeführt oder u.U. abgeschlossen werden. Daraus resultiert ein entsprechend großer Zeitbedarf.

Wiederum liegt aber auch die Genauigkeit der pH-Wert Messungen - selbst unter optimalen Bedingungen und bei entsprechender Kalibrierung - im allgemeinen nur bei ca. +/- 0,05 pH-Wert Einheiten. Der über den gesamten Zeitraum der Abfüllung zu gewährleistende pH-Wert muss zwischen 7,2 und 7,3 pH-Wert-Einheiten betragen. Da festzustellen ist, dass der pH-Wert zu höheren Werten hin im Laufe der Abfüllzeit driftet, ist der einzustellende Zielwert der niedrigste zulässige Wert von 7,2 pH-Wert-Einheiten.

Der pH-Wert ist letztlich auf Grund dieser Zusammenhänge als Regelgröße für die Kohlendioxidzugabe ungeeignet.

Die Annäherung an den einzustellenden Endzustand während des Begasungsvorganges erfolgte dementsprechend bei bekannten Verfahren nicht zielführend. Der Prozess war nur iterativ durchführbar.

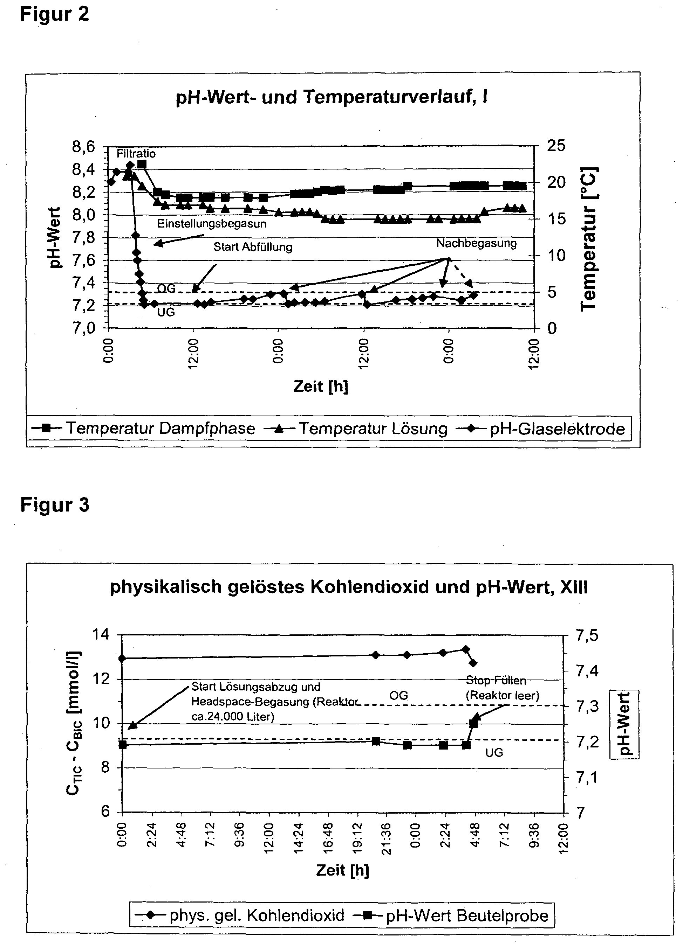

Gemäß der vorliegenden Erfindung ergibt sich dagegen eine reproduzierbare Einstellung des pH-Wertes vor Abfüllung hinsichtlich der Kohlendioxidbegasung unter Berücksichtigung des thermodynamischen Gleichgewichtszustandes und des Absorptionsverhaltens im System $CO_2$-$NaHCO_3$-$H_2O$, (2) in Abb. 2-1

Über den Zeitraum der Begasung ist das Ventil V2 geschlossen, so dass kein Kohlendioxid in die Umgebung abgegeben werden kann. Es ist die Möglichkeit zur Aufstellung einer $CO_2$ -Bilanz gegeben. Dabei wird hinsichtlich der Bilanz nur das im sterilen Reaktor zugegebene Kohlendioxid verstanden, d.h. ohne das bereits durch die Zugabe von Bicarbonat während des Ansatzvorganges durch Reaktion entstandene Kohlendioxid. Wiederum ist über das Rückschlagventil V3 ein etwaig notwendiger Zufluss von Umgebungsluft über den Sterilfilter gewährleistet. Damit kann auch dem möglichen Entstehen eines Unterdruckes durch Abkühlung entgegengewirkt werden (hohe Temperatur des Behälter nach Sterilisation).

Ein Austreten von Kohlendioxid wird somit verhindert.

Für den relevanten Temperatur- und Druckbereich der anzusetzenden Lösung muss die Güte der Annäherung an den Phasengleichgewichtszustand des Systems ermittelt werden. Die thermodynamischen Gleichgewichtsbedingungen besagen, dass Temperatur, Druck und die chemischen Potentiale in allen Phasen gleich sind.

Es ist für den einzustellenden pH-Wert die zusätzlich zu absorbierende Kohlendioxidmenge zu bestimmen. Für den gesuchten Druck- und Temperaturbereich ergeben sich auch die zugehörigen Werte der Kohlendioxidkonzentration in der Gas- und Flüssigphase und des Partialdruckes von Kohlendioxid der gasförmigen Phase gemäß den Gleichgewichtsbedingungen.

Zur Ermittlung der entsprechenden Daten und letztlich zur Abstimmung des Produktionsprozesses auf die theoretischen Daten, können sowohl Ergebnisse von Phasengleichgewichtsberechnungen dienen, als auch Messungen der Lösung hinsichtlich pH-Wert, Druck, Temperatur, Konzentration, $CO_2$-Verbrauch und TIC (Total Inorganic Carbon).

Um den thermodynamischen Zustand des Systems im Produktionsprozess zu erfassen, bietet es sich an, folgende Größen zu bestimmen:
Temperatur in Flüssig- und Dampfphase, Gesamtdruck bzw. Partialdruck von $CO_2$ im Kopfbereich des Reaktors, Kohlendioxidanteil in Dampf- und Flüssigphase. Die Bestimmung der Partialdruckänderungen von $CO_2$ lässt sich durch Bestimmung des Gesamtdruckes zu verschiedenen Zeitpunkten umsetzen, da ein Austreten von $CO_2$ unterbunden ist und ein etwaiger Druckanstieg beim Begasen daher nur durch $CO_2$ hervorgerufen wird.

Abhängig vom Chargenauftrag kann ebenso in diesem Fall anhand der Rezeptur und der Lösungsmenge mittels des Prozessleitsystems die zuzugebende Kohlendioxidmenge bestimmt und über eine Massendurchflussbestimmung von Kohlendioxid die Zufuhr begrenzt beziehungsweise gestoppt werden.

Messungen der vorgenannten Prozessgrößen liefern dann eine Aussage über die Güte der Annäherung an den Gleichgewichtszustand. Ein iteratives Vorgehen mit unzähligen zeitaufwendigen pH-Wert Messungen erübrigt sich.

➤ Kohlendioxidbedarf überschlägig errechnet:

Es soll hier eine überschlägige Berechnung des Kohlendioxidbedarfs anhand des Massenwirkungsgesetzes unter Verwendung von Literaturwerten für die Dissoziationskonstante $K_S$, bzw. den $pK_S$ - Wert, durchgeführt werden um einen Vergleich bzw. eine Einschätzung diesbezüglicher Ergebnisse gegenüber dem realen Fall zu erlangen.

Für die Reaktionsgleichungen gilt:

$$H_2O + CO_2 \rightleftarrows H_2CO_3$$

$$H_2CO_3 \rightleftarrows HCO_3^- + H^+$$

$$H_2O + CO_2 \rightleftarrows HCO_3^- + H^+$$

Die Bildung von $CO_3^{2-}$ (Carbonation) kann in dem betrachteten pH-Wert - Bereich vernachlässigt werden.

Es gilt gemäß obigen Ausführungen für den Gleichgewichtszustand:

$$K_C = \frac{(C_C)^{vC} \cdot (C_D)^{vD}}{(C_A)^{|v_A|} \cdot (C_B)^{|v_B|}} = \frac{[HCO_3^-] \cdot [H^+]}{[H_2O] \cdot [CO_2]}$$

mit $K_C$ als Gleichgewichtskonstante.

Aufgrund des großen Überschusses an Wasser kann vereinfachend angenommen werden:

$$K_C = \frac{[HCO_3^-] \cdot [H^+]}{[CO_2]}$$

mit $[HCO_3^-]$ = 70 mmol/l = 0,07 mol/l gegeben aus der "Mutterrezeptur" bzw. Herstellanweisung

Nach Holleman und Wiberg beträgt die Umsetzung von Kohlendioxid mit Wasser zu Kohlensäure $H_2CO_3$ jedoch nur etwa 0,2 %.

Diese als freie Säure nicht isolierbare Kohlensäure ist theoretisch eine mittelstarke Säure mit einer Dissoziationskonstanten $K_S$. $H^+$ steht dabei in allen Formeln für das tatsächlich vorliegende Hydroniumion $H_3O^+$.

$$K_S = \frac{[HCO_3^-] \cdot [H^+]}{[H_2CO_3]} \qquad 3 \times 10^{-4}$$

bzw. $pK_S$ = 3,88

Da jedoch ca. 99,8 % des gelösten Kohlendioxids nicht als $H_2CO_3$, sondern als hydratisiertes $CO_2$ vorliegen, wirkt die Gesamtlösung als schwache Säure.

Berücksichtigt man nun den undissoziierten Säureanteil, so kann man eine sogenannte "scheinbare Dissoziationskonstante" angeben und somit den eigentlichen Charakter der Lösung eher beschreiben:

$$K_S = \frac{[HCO_3^-] \cdot [H^+]}{[H_2CO_3 + CO_2]} = 4,5 \times 10^{-7} \qquad \text{bzw. } pK_S = 6,35$$

es gilt weiterhin:

$$pK_S = -lg\, K_S$$

$pK_S = 6,35$ [nach Hollemann und Wiberg]

$K_S = 10^{-6,35}$

Der pH-Wert der unbegasten Lösung im Reaktor (nach Filtration) beträgt gemäß den Messungen der Versuchsreihen etwa 8,5 pH-Wert Einheiten, der pH-Wert der fertig eingestellten Lösung nach Begasung 7,2. Dementsprechend kann für die Protonenkonzentration nach Definition des pH-Wertes angegeben werden:

unbegast: $[H^+] = 10^{-8,5}$ mol/l
begast: $[H^+] = 10^{-7,2}$ mol/l

Für die Kohlendioxidkonzentration ergibt sich nun aus obiger Beziehung:

$$[CO_2] = \frac{[HCO_3^-] \cdot [H^+]}{K_S}$$

id mit obigem Wert für $pK_S = 6,35$ folgt:
unbegast:

$$[CO_2] = \frac{0,07\ mol/l \cdot 10^{-8,5}\ mol/l}{10^{-6,35}\ mol/l} \cdot 10^{-3}\ mol/l$$

begast:

$$[CO_2] = \frac{0,07\ mol/l \cdot 10^{-7,2}\ mol/l}{10^{-6,35}\ mol/l} \cdot 10^{-3}\ mol/l$$

Dementsprechend ergibt sich als Differenz zwischen Anfang und Ende der Begasung für die Änderung der Kohlendioxidkonzentration:

$$[CO_2]_{Ende} - [CO_2]_{Anfang} = 0,008321\ mol/l$$

Mit einem Molekulargewicht für Kohlendioxid von 44,011 g/mol [Fa. Linde Gas], erhält man damit einen Kohlendioxidbedarf für 1 Liter Lösung:

$$m_{CO2} = M_{CO2} \cdot [CO2] = 44{,}011\ g/\,mol \cdot 0{,}008321\ mol/\,l = 0{,}366216\ g/\,l$$

bzw. : 8,789 kg Kohlendioxid/ 24000 Liter

Obige Rechnung berücksichtigt hinsichtlich der Berechnung des Volumens nur die 24m³ Lösungsmenge, der gegebene Reaktor weist allerdings einen Inhalt von 26,8 m³ auf. Aus den theoretischen Berechnungen zum Phasengleichgewicht (siehe Anlage B) kann entnommen werden, dass die Dampfphase bei einer Lösungsmenge von 24 m³ etwa 1,054 kg Kohlendioxid aufweist. Um die Ergebnisse pauschal miteinander vergleichen zu können, wird obiges Ergebnis um diesen Wert korrigiert und man erhält nach Addition beider Werte einen Kohlendioxidbedarf für 24.000 Liter Lösung im Reaktor von :

$$m1 \quad + \quad m2 \quad = \quad m_{ges}$$

8,789 kg + 1,054 kg = 9,843 kg Kohlendioxid/ 24000 Liter Lösung im Reaktor

In der idealisierten Rechnung mittels des Massenwirkungsgesetzes ist insbesondere nicht die Anwesenheit von Natriumhydrogencarbonat berücksichtigt und der hieraus resultierenden Kohlendioxidmenge.

Es wurden daher Vergleichsmessungen hinsichtlich des tatsächlich zur Einstellung des pH-Wert benötigten $CO_2$-Menge durchgeführt.

Dabei wird die Menge an zuzugebendem Kohlendioxid bestimmt, um zielgerichtet die Lösung einstellen zu können.

Es besteht damit die Möglichkeit entsprechend den spezifischen Kohlendioxidmengen für die Behälter bezüglich der einzelnen Füllhöhen mittels des Prozessleitsystems - anhand der jeweils vorgewählten Lösungsmenge - die entsprechende zu dosierende Kohlendioxidmenge selbsttätig errechnen zu lassen und bei erreichtem dosierten Zielwert des Kohlendioxides, die Gaszufuhr automatisiert zu stoppen. Damit ist auch den Einflussfaktoren der unterschiedlichen Füllhöhen Rechnung getragen. Eine nachfolgende pH-Bestimmung kann dann den zu erreichenden pH-Wert von 7,2 bestätigen.

Absolute Mengen betrachtet, ergibt sich ein etwas niedrigerer Bedarf im Falle höherer Lösungsmengen.

Vereinfacht man die Betrachtung ohne Unterscheidung der Art der Begasung und der Lösungsmengen, so erstreckt sich der absolute Bereich der eingesetzten Kohlendioxidmengen von 10,25 kg bis 12,9 kg.

Dieses kann man nun den Ergebnissen der theoretischen Berechnungen zum Phasengleichgewicht gegenüberstellen.

Man benötigt eine bestimmte Kohlendioxidmenge in der Flüssigkeit und in der Dampfphase. Sinkt der Füllstand so tritt Kohlendioxid aus der Flüssigkeit in die Dampfphase. Je nach Füllhöhe variiert der Anteil von Kohlendioxid in der Flüssigphase bzw. der Dampfphase.

Für den Gleichgewichtszustand ergeben für vorliegendes System die Berechnungen einen mittleren Wert von ca.10,5 kg Kohlendioxid.

Es lässt sich damit insgesamt gesehen mit obigen Werten eine gute Näherung an diesen theoretischen, für reale Zusammenhänge nur näherungsweisen erreichbaren Zustand finden.

Versuchsauswertungen haben gezeigt, dass zwar die Begasungszeit über die Versuchsreihen erheblich variieren kann, jedoch die einzusetzende Kohlendioxidmenge davon weitestgehend unbeeinflusst ist.

Einsparungen ergeben sich in diesem Zusammenhang durch die gezielte Vorgabe einer zu dosierenden Kohlendioxidmenge hinsichtlich einem geringen Personalbedarf und einer erhöhten Anlagenverfügbarkeit der Produktion der Ansatzlinie. Ausgehend von etwa dreimaligem Stoppen des Begasungsmodus und entsprechendem Probenzug, Kalibrierung der pH-Messung im Labor, Gegenmessung und erneutem Start der Begasung, ist eine pH-Wert-Bestimmung nun nur einmalig nach dem Erreichen des zielführend angefahrenen Endzustandes zur Bestätigung des erreichten Zustandes erforderlich.

Bei einem Zeitbedarf je oben beschriebener Aktion von etwa 20 Minuten ergibt sich eine Einsparung des Personalbedarfs von etwa 40 Minuten je Ansatz. Gleichermaßen eine erhöhte Anlagenverfügbarkeit, Verfügbarkeit der Laborgeräte und Einsparung von Verbrauchsmaterialien.

Drift des pH-Werts über den Zeitraum der Abfüllung bei dem Verfahren gemäß dem Stand der Technik:
Das Abfüllen der Lösung bedingt eine stetige Abnahme des Füllstandes des sterilen Reaktors über einen Zeitraum von ca. vierzig Stunden. Über diesen Prozesszeitraum ist festzustellen, dass der pH-Wert der Lösung bei bekannten Verfahren sukzessive zunimmt und einen oberen Grenzwert von pH 7,3 überschreitet.

In diesem Zustand wird üblicherweise die Abfüllung auf Grund der installierten Inline pH-Wert Sonde durch automatisiertes Schließen der Füllventile sofort abgebrochen.

Die Lösung wird nun anhand Erfahrungswerten des jeweiligen Bedienpersonals einige Minuten mit Kohlenddioxid nachbegast.

Die Begasung wird nach einer gewissen Zeit beendet und mittels Probe und LaborpH-Meter der erzielte pH-Wert mehrmals bestimmt. Diese Vorgehensweise wird entsprechend oft wiederholt bis zum Erreichen eines pH-Wertes zwischen 7,2 und 7,3. Dabei wird - entsprechend der ursprünglichen Einstellung nach dem Ansetzen der Lösung - wieder eine Einstellung an dem unteren Grenzwert 7,2 angestrebt, da weiterhin ein Ansteigen des pH-Wertes bei sinkendem Füllstand erfolgt.

Die installierte Inline-Sonde wird gegebenenfalls anhand der pH-Wert Messung mittels Labor pH-Meter über ihren Messumformer an den aktuellen Wert angepasst.

Der Produktionsausfall, beziehungsweise Stillstand einer solchen Nachbegasungsaktion, beläuft sich jeweils auf ca. 45 Minuten. Der zeitliche Verlauf des Auftretens ist ebenso abhängig von der Güte der ursprünglichen Anfangseinstellung und kann im Rahmen eines Abfüllzyklus einer Charge - selbst bei optimaler Einstellung der Lösung nahe der Untergrenze pH 7,2 - erfahrungsgemäß etwa viermal erforderlich werden.

Figur 2 zeigt den Status Quo, bzw. die Anwendung des bisherigen Begasungs- und Abfüllprozesses hinsichtlich der pH-Wert-Entwicklung.

Die vorliegende Erfindung ermöglicht dagegen eine Stabilisierung des pH-Wertes während der Abfüllung durch Nachführung von Kohlendioxid entsprechend den Zustandsgrößen im thermodynamischen Gleichgewicht in Abhängigkeit des Füllstandes des Behälters.

Während des Abfüllprozesses wird das entstehende zusätzliche Volumen durch über den sterilen Luftfilter nachströmende Raumluft ersetzt. Dadurch sinkt die Kohlendioxid-konzentration im Gasraum des Reaktors gegenüber der anfänglichen Situation.

Es stellt sich damit ständig erneut ein Gleichgewichtszustand näherungsweise zwischen den beiden Phasen ein. Dieses führt zum Austreten von Kohlendioxid aus der Lösung in den Dampfphase, wodurch der pH-Wert der Lösung ansteigt und auch nach einer gewissen Zeit über die obere Grenze von 7,3 pH-Wert-Einheiten gelangt.

Das Rückschlagventil V3 ermöglicht während dem Abfüllprozess beziehungsweise bei sinkendem Reaktorfüllstand ein Nachströmen von Raumluft, wodurch die Bildung eines Unterdruckes verhindert wird. Gleichfalls werden separat - in Abhängigkeit des gemessenen Füllstandes und der bekannten Kohlendioxidkonzentration der Dampfphase im Phasengleichgewicht - quasikontinuierlich Kleinmengen an Kohlendioxid über das Ventil V4 in den Gasraum zugegeben. Es wird auf Grund des Rückschlagventils und des während dem Abfüllzeitraum geschlossenen Luftfilterventils V2 das Ausströmen irgendwelcher Gasanteile unterbunden.

Die permanente Rückführung der in dem Abfüllkreislauf, beziehungsweise "großen Kreislauf", gefahrenen Lösungsmenge erfolgt über ein Tauchrohr, so dass ein unmittelbares Absorbieren von Kohlendioxid beim etwaigen Durchtreten des Flüssigkeitsstrahles durch die Dampfphase vermieden wird.

Der thermodynamische Zustand zwischen Dampf- und Flüssigphase, bzw. der anfangs eingestellte näherungsweise Gleichgewichtszustand im Reaktor bleibt nahezu erhalten und einer Desorption von Kohlendioxid beziehungsweise einem Ansteigen des pH-Wertes wird entgegengewirkt.

Zusammenfassend lässt sich feststellen, dass durch diese Vorgehensweise das Entstehen eines Gradienten des pH-Wertes über den Abfüllzeitraum der jeweiligen Charge (ca. 24.000 Beutelsysteme) unterbunden wird, da keine Drift des pH-Wertes auftritt.

Da sich dadurch pro abgefüllter Charge etwa vier Nachbegasungsaktionen erübrigen, kann ein Produktionsausfall von ca. 3 Stunden pro Charge vermieden werden.

Alternatives Lösungskonzept: Stabilisierung des pH-Wertes während der Abfüllung durch Nachführung von Kohlendioxid entsprechend den Zustandsgrößen im thermodynamischen Gleichgewicht in Abhängigkeit der Menge der nachströmenden Raumluft.

Anlehnend an das soeben gezeigte Vorgehen ist denkbar, das Nachführen von Kohlendioxid nur dann mittels SPS-Steurung durchzuführen, wenn gleichfalls ein Einströmen bzw. Einsaugen von Raumluft - auf Grund der Abnahme des Füllstandes und des entstehenden Unterdruckes - über den Sterilfilter gegeben ist (V2 permanent offen). Zur Feststellung dieses Zustandes ist eine entsprechende im Feinbereich funktionierende Durchflussmessung im Bereich des Filters vorzusehen (FIC1), welche über das Ventil V1 die Zuführung von Kohlendioxid steuert.

Die Zugabe von $CO_2$ soll dabei gemäß dem Molanteil im theoretisch zu bestimmenden Gleichgewichtszustand erfolgen. Der Molanteil muss dabei über den Abfüllzeitraum gleichbleibend sein, da eben pH-Wert, Temperatur, Druck, Zusammensetzung des Lösungssystems auch aufrechterhalten werden sollen. Die Kohlendioxidzufuhr wäre darüber hinaus nur bestimmt durch die Menge der einströmenden Raumluft und dementsprechend durch die Abfüllgeschwindigkeit der Füllereinheiten beziehungsweise Beutelsysteme.

Bei Ausführung des Gaszufuhrventils V1 als Regelventil kann daher die Menge der einströmenden Raumluft gleichfalls die Sollwertvorgabe für das Gaszufuhrventil V1 liefern um somit das vorgegebene Verhältnis von Kohlendioxid zu Raumluft in der Dampfphase des Behälters aufrechtzuerhalten. Das System reagiert damit unmittelbar auf unterschiedlich schnelle Abfüllvorgänge. Die Zufuhr von Kohlendioxid erfolgt im Gegensatz zu den Ausführungen in obigem Fall kontinuierlich, genau über den Zeitraum, in dem auch eine Zufuhr von Raumluft gegeben ist. Dadurch erfolgt die Einstellung der Zusammensetzung der Dampfphase des Reaktors zielorientiert und führt unmittelbar zu stabilen Verhältnissen.

Die Lösung der Nachführung von Kohlendioxid gemäß der in die Anlage nachströmenden Raumluftmenge ist jedoch problematisch, da die Gasdichtigkeit des Reaktorsystems hinsichtlich angeschlossener Rohrleitungen gegeben sein muss. Deshalb ist eine Steuerung auf Grundlage der Änderung des Füllstandes vorzuziehen.

Die nachgeführte Menge wird dabei entsprechend der Geschwindigkeit der Füllstandabnahme dem System geregelt zugeführt und berücksichtigt die Zusammensetzung im theoretisch ermittelten Phasengleichgewichtszustand, bei dem hier betrachteten Lösungssystem mit etwa 20,5 Vol% Kohlendioxid in der Dampfphase.

Interessant ist die gute Reproduzierbarkeit der nachgeführten Kohlendioxidmenge in Höhe von 11,15 kg (Figur 3) gegenüber dem Einstellungsverbrauch von 12,9 kg und der aufgrund theoretischer Berechnungen ermittelten Kohlendioxid-menge im Falle des Phasengleichgewichts für den Fall des gerade entleerten Reaktors (10,09 kg). Unterstellt man einen ideal verlaufenden konstanten pH-Wert und die Aufrechterhaltung eines Phasengleichgewichtszustandes, so müsste noch der Anteil der schon im Reaktor in der Dampfphase befindlichen Kohlendioxidmenge bei 24000 Liter Inhalt zugerechnet werden. Nach den errechneten Werten entspräche diese Menge etwa 1,05 kg Kohlendioxid. Hinzu käme eine vernachlässigbare Menge auf Grund des in der angesaugten Raumluft vorhandenen Kohlendioxides.

Da die Regelung auf der im Reaktor gemessenen Füllstandsabnahme basiert, reagiert der Reaktor in diesem Sinne mit obiger Regelung selbsttätig und ohne weiteres manuelles Zutun auf Veränderungen der örtlich weit entfernten Produktionslinie der Abfüllung.

In den weiteren Ausführungen ist auch die Regelungsstruktur näher beschrieben. Im folgenden soll daher ohne weitere Erläuterungen das Ergebnis nochmals anhand der Kurvendarstellungen der pH-Wert-Messung wiedergegeben werden.

Das hier erarbeitete Verfahren wird in diesem Zusammenhang als sogenanntes "Headspace-Verfahren" bezeichnet.

Die Darstellung in Figur 3 zeigt einen bis unmittelbar vor Beendigung der Abfüllung konstant blei-benden pH-Wert Verlauf (im Gegensatz zur Auswertung gemäß Figur 2).

Es sei an dieser Stelle darauf hingewiesen, dass auch eine Erhöhung der zulässigen pH-Wert Grenze nicht vor den notwendigen Nachbegasungsaktionen bewahrt. Der im Rahmen der Versuchsreihe I wiedergegebene Kurvenverlauf (siehe Figur 2) des pH-Wertes verdeutlicht das schnelle Ansteigen zu höheren Werten hin, die Steigung der Kurve zeigt, dass beispielsweise eine Anhebung der zulässigen IPK-Obergrenze auf pH 7,4 keine Lösung des Problems darstellen kann.

Verbesserungsmöglichkeiten durch entsprechende Gestaltung des Tauchrohres (Anordnung in der Nähe des Behälterauslaufes) ergeben sich im Falle des Leerfahrens des Reaktors. Dort ist ein pH-Wert Anstieg kurzzeitig auf Grund des Austritts der abgetauchten Rückführleitung aus der Flüssigkeitsoberfläche gegeben (siehe Figur 3). Bislang wird dieser Zustand bedingt durch den Produktionsablauf der Abfülllinie nie erreicht, da vor dem Leerfahren des Reaktors die Restmenge von wenigen Litern verworfen wird und der Reaktor mittels separater Ansteuerung entleert wird.

Da das Leerfahren des Reaktors jedoch mit einer automatischen Umschaltung von einem Reaktor auf den anderen verbunden wäre, könnte damit eine anzustrebende halbkontinuierliche Fahrweise realisiert werden. Selbst bei gegebenem Anstieg des pH-Wertes liegt dieser noch im zulässigen Bereich, d.h. kleiner 7,3 pH-Wert Einheiten. Dieses wird letztlich durch das vorangegangene Konstanthalten des pH-Werts mit pH = 7,2 ermöglicht.

Es ergibt sich mit der Umsetzung dieses Verfahrens eine Reduzierung des Personalaufwandes auf Grund wegfallender Nachbegasungsaktionen von etwa 45 Minuten pro Unterbrechung bei etwa 2 bis 4 Unterbrechungen je nach Lösungsmenge beziehungsweise Abfülldauer über den Zeitraum der Abfüllung. Je nach Auftragslage, beziehungsweise Marktnachfrage nach verschiedenen Beutelgrößen eines Lösungstypes, ergibt sich eine jährlich kalkulierbare abzufüllende Lösungsmenge. Die Nachfrage des Vertriebes aus verschiedenen Ländern steuert die zu gewissen Zeitpunkten benötigte Beutelstückzahl und gibt damit die Ansatzgrößen beziehungsweise Reaktorfüllmengen vor.

Im Gegensatz zu den oben aufgezeigten zwei- bis viermaligen Unterbrechungen der Produktion zu je etwa 45 Minuten werden mit der Anwendung des erfindungsgemäßen Verfahrens beim betrachteten Lösungstyp die bisherigen erheblichen Produktionsausfallzeiten vermieden. Darüber hinaus minimiert sich entsprechend der Personalaufwand zur Durchführung von Nachbegasungsaktionen.

Der entstehende Mehraufwand durch die Installation eines Regelventils, der zugehörigen Verrohrung und einer Durchflussmengenmessung für Kohlendioxid zuzüglich dem Mehrbedarf an Kohlendioxid (11,15 kg, Figur 3) ist gegenüber der Ersparnis marginal.

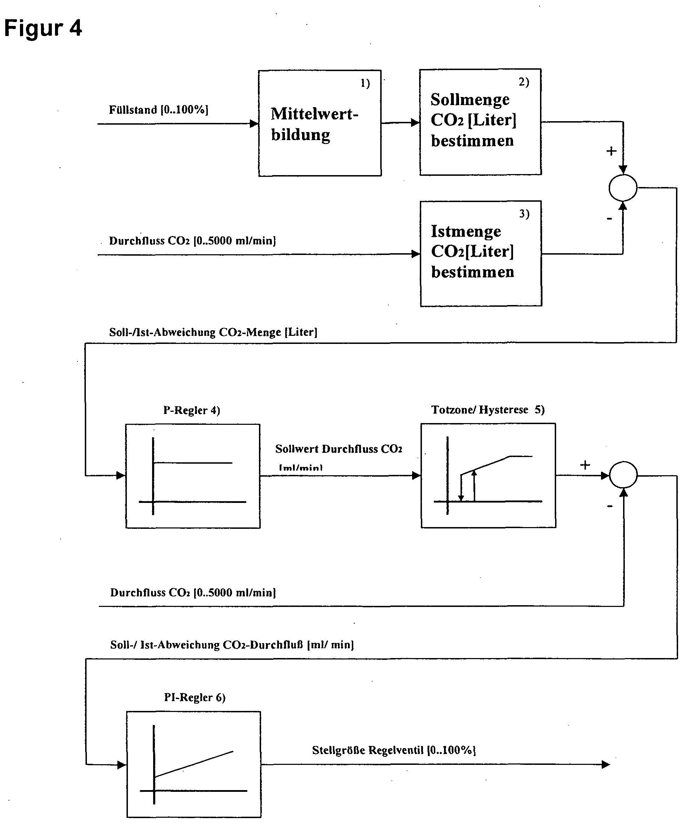

Figur 4 zeigt den schematischen Regelungsablauf ausgehend vom Eingangssignal (Füllstand 0 % bis 100 %) bis hin zur Stellgröße (0 % bis 100 %) des Regelventils.

Die Umsetzung erfolgte auf einer speicherprogrammierbaren Steuerung S5 - 115 U CPU 944 B.

Die im Regelungsschema in Figur 4 angeführten Nummern 1) bis 6) sollen im folgenden kurz erläutert werden:

Zu 1)	Mittelwertbildung aus 100 Werten (ein neuer Wert pro Sekunde).

Zu 2) 1 % Füllstandsabnahme entspricht 250 Liter abgefüllte Lösung, beziehungsweise 250 Liter Volumenzunahme. 20,5 % (Kohlendioxid; aus Phasengleichgewichtsbetrachtungen) von 250 Liter ergibt 51,25 Liter zuzugebendes Kohlendioxid pro 1% Füllstandsabnahme.

Zu 3)Istmenge durch Integration des Durchflusses über die Zeit.

Zu 4) P-Regler zur Bewertung des Soll-/Istvergleichs der Kohlendioxidmenge, beziehungsweise als Verstärkungsfaktor um möglichst stabiles Regelverhalten zu erreichen (Ausgangsgröße entspricht Solldurchfluss Kohlendioxid) Verstärkungsfaktor = 100, d.h. pro Liter Soll-/ Istabweichung der CO2-Menge wird der Sollwert für den Durchfluss um 100 ml/ min erhöht.

Zu 5) Berücksichtigung einer Totzone/ Hysterese um in einem sinnvollen bzw. zulässigen Arbeitsbereich von 1 bis 5 L/ min zu messenden Kohlendioxid zu bleiben.
Sollwert CO2 - Durchfluss wird bei Anstieg bis 1000 ml/ min, bei Abfall ab 900 ml/ min auf 0 ml/ min gesetzt (Verhinderung von "Schleichmengen", bzw. fehlen beispielsweise weniger als 10 Liter CO2, dann würde das Regelventil zugefahren bleiben (10 Liter Eingangsgröße x Verstärkung 100 = 1000 ml/ min Ausgangsgröße).
Ab 5000 ml/ min wird der Sollwert auf 5000 ml/ min gesetzt, bzw. "eingefroren" (maximaler Messbereich des Durchflussmessers).

Zu 6) PI-Regler des Regelventils zur "Ausregelung", bzw. Angleichung des Soll-/ Ist-Zustandes. Um ein möglichst stabiles Regelverhalten zu erzielen, wird eine Soll-/ Istabweichung mit 5 % bewertet.

Die vorliegende Erfindung zeigt damit ein Verfahren, welches durch Nachführung von Kohlendioxid in die Dampfphase von Gas-Flüssig Reaktoren einen zuvor eingestellten Systemzustand der flüssigen Phase hinsichtlich eines konstant zu haltenden pH-Wertes gewährleistet. Das Verfahren wird in diesem Zusammenhang als "Headspace-Verfahren" bezeichnet. Dabei verhindert die Aufrechterhaltung eines angenäherten Phasengleich-gewichtszustandes das Ausgasen von Kohlendioxid aus der gegebenen flüssigen Phase.
Durch Umsetzung des "Headspace-Verfahrens", kann der Personalaufwand reduziert werden und insbesondere die Anlagenverfügbarkeit der betreffenden Produktionslinie erhöht werden.
Ebenso kann die vorliegende Erfindung bei der Herstellung bzw. dem Abfüllen weiterer Lösungen vorteilhaft eingesetzt werden, z. B. bei einer mit Kohlendioxid zu begasenden Lösung mit geändertem Hydrogencarbonatgehalt und zugesetztem Natriumchlorid als Elektrolyten.
Entsprechend der bestimmten theoretischen Kohlendioxidkonzentrationen des thermodynamischen Gleichgewichts, wird für dieses Lösungssystem analog eine Lösungseinstellung vorgenommen und Kohlendioxid in den Reaktorkopf nachgeführt.
Das erfindungsgemäße Headspace-Verfahren kann ebenso bei weiteren Medizinproduktelösungen oder Arzneimittellösungen vorteilhaft eingesetzt werden, welcher mit Kohlendioxid begast werden müssen.

## Patentansprüche

1. Verfahren zum Konstanthalten eines pH-Werts einer medizinischen Flüssigkeit beim Ablassen aus einem Behälter, bei welchem während des Ablassens der medizinischen Flüssigkeit $CO_2$ und mindestens ein weiteres Gas oder Gasgemisch in den Behälter zuströmt,
**dadurch gekennzeichnet,**
**dass** eine Änderung des Füllstands (L1) der Flüssigkeit gemessen und anhand der Messung die Menge an $CO_2$, welche in den Behälter zugegeben wird, bestimmt wird.

2. Verfahren nach Anspruch 1, wobei eine Änderung des Gasraumvolumens im Behälter über der Flüssigkeit anhand der Änderung des Füllstands der Flüssigkeit bestimmt wird, und die zuzugebende Menge an $CO_2$ in Abhängigkeit von dieser Änderung des Gasraumvolumens bestimmt wird.

3. Verfahren nach einem der vorangegangenen Ansprüche, wobei eine Verbindung zwischen dem Gasraum des Behälters und der Umgebung vorgesehen ist, durch welche über einen Sterilfilter (F1) Umgebungsluft in den Gasraum zuströmen kann, wobei beim Ablassen der Flüssigkeit Umgebungsluft über den Sterilfilter (F1) nachströmt, so dass bei einer Änderung des Füllstands (L1) der Flüssigkeit der Druck im Gasraum dem Umgebungsdruck selbsttätig angeglichen wird.

4. Verfahren nach Anspruch 3, wobei das $CO_2$ in einer solchen Menge zugegeben wird, dass ein rechnerisch bestimmter mittlerer $CO_2$-Volumenanteil im Gasraum konstant bleibt.

5. Verfahren nach Anspruch 3 oder 4, wobei die zuzugebende Menge an $CO_2$ auf Grundlage eines rechnerisch ermittelten chemisch-physikalischen Gleichgewichts zwischen der Flüssigkeit und dem darüberliegendem Gasraum bestimmt wird.

6. Verfahren nach einem der vorangegangenen Ansprüche, wobei die Bestimmung der zuzugebenden Menge an $CO_2$ ohne eine Messung des pH-Werts der Flüssigkeit erfolgt.

7. Verfahren nach einem der vorangegangenen Ansprüche, wobei die Bestimmung der zuzugebenden Menge an $CO_2$ ohne eine Messung der $CO_2$-Konzentration im Gasraum erfolgt.

8. Verfahren nach Anspruch 1, wobei das $CO_2$ und das mindestens eine weitere Gas oder Gasgemisch während des Ablassens der medizinischen Flüssigkeit oberhalb des Flüssigkeitsspiegels in den Gasraum zuströmt.

9. Verfahren nach einem der vorangegangenen Ansprüche, wobei vor dem Ablassen der Flüssigkeit eine vorherbestimmte Menge an $CO_2$ in die Flüssigkeit eingedüst wird.

10. Verfahren nach einem der vorangegangenen Ansprüche, wobei die Flüssigkeit eine Bikarbonat-Lösung ist.

11. Vorrichtung zum Konstanthalten eines pH-Werts einer medizinischen Flüssigkeit beim Ablassen aus einem Behälter, wobei der Behälter einen Ablauf (V6) für die medizinische Flüssigkeit, mindestens einen Zulauf (V5) für $CO_2$ sowie einen Zulauf (V3) für mindestens ein weiteres Gas oder Gasgemisch aufweist, mit einem Messwertgeber zur Bestimmung des Füllstandes (L1) im Behälter und mit einer elektronischen Steuerung zur Bestimmung und automatischen Zuführung einer $CO_2$-Menge zur anfänglichen Lösungseinstellung mittels Gas-Flüssigdüsen (I1) und/oder während des Ablassens der medizinischen Flüssigkeit, wobei eine Änderung des Füllstands (L1) der Flüssigkeit gemessen und anhand der Messung die Menge an $CO_2$, welche in den Behälter zugegeben wird, bestimmt wird.

12. Vorrichtung nach Anspruch 11, wobei der Messwertgeber den Füllstand (L1) im Behälter über eine Mikrowellen- bzw. Radarmessung bestimmt.

13. Vorrichtung nach Anspruch 11 oder 12, mit einer Verbindung (V3) zwischen dem Gasraum des Behälters und der Umgebung, durch welchen über einen Sterilfilter (F1) Umgebungsluft in den Gasraum zuströmen kann.

14. Vorrichtung nach einem der Ansprüche 11 bis 13 zur Durchführung eines Verfahrens gemäß einem der Ansprüche 1 bis 10.

**Claims**

1. A method for the keeping constant of a pH of a medical liquid when draining from a container in which $CO_2$ and at least one further gas or gas mixture flow into the container during the draining of the medical liquid,
**characterized in that**
a change in the filling level (L1) of the liquid is measured and the amount of $CO_2$ which is added into the container is determined on the basis of the measurement.

2. A method in accordance with claim 1, wherein a change in the gas space volume over the liquid in the container is determined on the basis of the change in the filling level of the liquid, and the quantity of $CO_2$ to be added is determined in dependence on this change in the gas space volume.

3. A method in accordance with one of the preceding claims, wherein a connection is provided between the gas space of the container and the environment through which ambient air can flow into the gas space via a sterile filter (F1), with ambient air flowing in over the sterile filter (F1) when draining the liquid so that, on a change in the filling level (L1) of the liquid, the pressure in the gas space is automatically approximated to the ambient pressure.

4. A method in accordance with claim 3, wherein the $CO_2$ is added in such a quantity that a mean $CO_2$ volume portion in the gas space determined by calculation remains constant.

**5.** A method in accordance with claim 3 or 4, wherein the quantity of $CO_2$ to be added is determined on the basis of a chemical-physical equilibrium determined by calculation between the liquid and the gas space disposed above it.

**6.** A method in accordance with one of the preceding claims, wherein the determination of the quantity of $CO_2$ to be added takes place without a measurement of the pH of the liquid.

**7.** A method in accordance with one of the preceding claims, wherein the determination of the quantity of $CO_2$ to be added takes place without a measurement of the $CO_2$ concentration in the gas space.

**8.** A method in accordance with claim 1, wherein the $CO_2$ and the at least one further gas or gas mixture flows into the gas space above the liquid level during the draining of the medical liquid.

**9.** A method in accordance with one of the preceding claims, wherein a previously determined quantity of $CO_2$ is injected into the liquid by nozzle before the draining of the liquid.

**10.** A method in accordance with one of the preceding claims, wherein the liquid is a bicarbonate solution.

**11.** An apparatus for the keeping constant of a pH of a medical liquid when draining from a container, with the container having an outlet (V6) for the medical liquid, at least one inlet (V5) for $CO_2$ as well as an inlet (V3) for at least one further gas or gas mixture, comprising a transducer for the determination of the filling level (L1) in the container and an electronic control for the determination and automatic supply of a $CO_2$ quantity for the initial solution setting by means of gas/liquid nozzles (I1) and/or during the draining of the medical liquid, wherein a change in the filling level (L1) of the liquid is measured and the quantity of $CO_2$ which is added into the container is determined on the basis of the measurement.

**12.** An apparatus in accordance with claim 11, wherein the transducer determines the filling level (L1) in the container via microwave measurement or radar measurement.

**13.** An apparatus in accordance with claim 11 or 12, comprising a connection (V3) between the gas space of the container and the environment by which ambient air can flow into the gas space over a sterile filter (F1).

**14.** An apparatus in accordance with one of claims 11 to 13 for the carrying out of a method in accordance with one of claims 1 to 10.

**Revendications**

**1.** Procédé pour maintenir constant le pH d'un liquide médical lorsqu'il s'écoule d'un récipient, dans lequel du $CO_2$ et au moins un autre gaz ou mélange gazeux affluent dans le récipient pendant l'écoulement du liquide médical, **caractérisé en ce que**
une modification du niveau de remplissage (L1) du liquide est mesurée et la quantité de $CO_2$ qui est ajoutée dans le récipient est déterminée à l'aide de la mesure.

**2.** Procédé selon la revendication 1, dans lequel une modification du volume de l'espace gazeux dans le récipient au-dessus du liquide est déterminée à l'aide de la modification du niveau de remplissage du liquide, et la quantité de $CO_2$ à ajouter est déterminée en fonction de cette modification du volume de l'espace gazeux.

**3.** Procédé selon l'une des revendications précédentes, dans lequel une liaison entre l'espace gazeux du récipient et l'environnement est prévue, par laquelle de l'air ambiant peut affluer dans l'espace gazeux par le biais d'un filtre stérilisant (F1), de l'air ambiant entrant par le biais du filtre stérilisant (F1) lors de l'écoulement du liquide, de telle sorte que, lors d'une modification du niveau de remplissage (L1) du liquide, la pression dans l'espace gazeux est automatiquement égalisée avec la pression ambiante.

**4.** Procédé selon la revendication 3, dans lequel le $CO_2$ est ajouté dans une quantité telle qu'une fraction volumique de $CO_2$ moyenne déterminée par voie de calcul reste constante dans l'espace gazeux.

**5.** Procédé selon la revendication 3 ou 4, dans lequel la quantité de $CO_2$ à ajouter est déterminée sur la base d'un équilibre physicochimique déterminé par voie de calcul entre le liquide et l'espace gazeux se trouvant au-dessus de celui-ci.

**6.** Procédé selon l'une des revendications précédentes, dans lequel la détermination de la quantité de $CO_2$ à ajouter est effectuée sans mesure du pH du liquide.

**7.** Procédé selon l'une des revendications précédentes, dans lequel la détermination de la quantité de $CO_2$ à ajouter est effectuée sans mesure de la concentration en $CO_2$ dans l'espace gazeux.

**8.** Procédé selon la revendication 1, dans lequel le $CO_2$ et l'au moins un autre gaz ou mélange gazeux affluent dans l'espace gazeux au-dessus du niveau de liquide pendant l'écoulement du liquide médical.

**9.** Procédé selon l'une des revendications précédentes, dans lequel, avant l'écoulement du liquide, une quantité prédéfinie de $CO_2$ est injectée dans le liquide.

**10.** Procédé selon l'une des revendications précédentes, dans lequel le liquide est une solution de bicarbonate.

**11.** Dispositif pour maintenir constant le pH d'un liquide médical lorsqu'il s'écoule d'un récipient, le récipient comportant une évacuation (V6) pour le liquide médical, au moins une arrivée (V5) pour du $CO_2$ ainsi qu'une arrivée (V3) pour au moins un autre gaz ou mélange gazeux, comprenant un capteur de mesure destiné à déterminer le niveau de remplissage (L1) dans le récipient et une commande électronique destinée à déterminer et à alimenter automatiquement une quantité de $CO_2$ pour le réglage initial de la solution au moyen de buses pour gaz et liquide (I1) et/ou pendant l'écoulement du liquide médical, une modification du niveau de remplissage (L1) du liquide étant mesurée et la quantité de $CO_2$ qui est ajoutée dans le récipient étant déterminée à l'aide de la mesure.

**12.** Dispositif selon la revendication 11, dans lequel le capteur de mesure détermine le niveau de remplissage (L1) dans le récipient par une mesure par micro-ondes ou par radar.

**13.** Dispositif selon la revendication 11 ou 12, comprenant une liaison (V3) entre l'espace gazeux du récipient et l'environnement, par laquelle de l'air ambiant peut affluer dans l'espace gazeux par le biais d'un filtre stérilisant (F1).

**14.** Dispositif selon l'une des revendications 11 à 13 destiné à exécuter un procédé selon l'une des revendications 1 à 10.

**Figur 1**

## Figur 2

**pH-Wert- und Temperaturverlauf, I**

Legend: —■— Temperatur Dampfphase —▲— Temperatur Lösung —◆— pH-Glaselektrode

## Figur 3

**physikalisch gelöstes Kohlendioxid und pH-Wert, XIII**

Legend: —◆— phys. gel. Kohlendioxid —■— pH-Wert Beutelprobe

# Figur 4

Füllstand [0..100%]

**Mittelwert-bildung** 1)

**Sollmenge CO₂ [Liter] bestimmen** 2)

Durchfluss CO₂ [0..5000 ml/min]

**Istmenge CO₂[Liter] bestimmen** 3)

+
-

Soll-/Ist-Abweichung CO₂-Menge [Liter]

P-Regler 4)

Totzone/ Hysterese 5)

Sollwert Durchfluss CO₂ [ml/min]

+
-

Durchfluss CO₂ [0..5000 ml/min]

Soll-/ Ist-Abweichung CO₂-Durchfluß [ml/ min]

PI-Regler 6)

Stellgröße Regelventil [0..100%]

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 491981 A1
- DE 2358759 A
- WO 2006090869 A1